(19)

Europäisches Patentamt

European Patent Office

Office européen des brevets

(11) **EP 2 048 222 A1**

(12) **EUROPEAN PATENT APPLICATION**

(43) Date of publication:
**15.04.2009 Bulletin 2009/16**

(21) Application number: **08017582.1**

(22) Date of filing: **07.10.2008**

(51) Int Cl.:
*C12M 1/22* (2006.01)          *C12M 1/34* (2006.01)
*G01B 11/06* (2006.01)          *G01N 21/41* (2006.01)

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(30) Priority: **10.10.2007 JP 2007264494**

(71) Applicant: **Olympus Corporation**
**Tokyo 151-0072 (JP)**

(72) Inventors:
• **Fukuda, Hiroshi**
  **Hino-shi**
  **Tokyo 191-0053 (JP)**

• **Kobayashi, Masayuki**
  **Hachioji-shi**
  **Tokyo 192-0023 (JP)**
• **Sakamoto, Takamitsu**
  **Hino-shi**
  **Tokyo 191-0041 (JP)**

(74) Representative: **von Hellfeld, Axel**
  **Wuesthoff & Wuesthoff**
  **Patent- und Rechtsanwälte**
  **Schweigerstrasse 2**
  **81541 München (DE)**

(54) **Culture vessel and cellular thickness measurement method**

(57)    To enable precise measurement of a cellular thickness distribution regardless of changes in the refractive index of a culture solution with the progress of culture, there is provided a culture vessel (1) made of a transparent material with a culture surface (1a) capable of culturing cells S in an adhesive manner on the bottom face, wherein a reference substance (2) having an already known refractive index and an already known thickness dimension is fixed to a part of the culture surface (1a).

## FIG. 1

**Description**

BACKGROUND OF THE INVENTION

1. FIELD OF THE INVENTION

The present invention relates to a culture vessel and a cellular thickness measurement method.

2. DESCRIPTION OF RELATED ART

Conventionally, as a method for extracting a cell image from a whole image including cultured cells, a method described in Japanese Unexamined Patent Application, Publication No. 2005-218379 has been known, for example.

This method is to conduct image subtraction using two contrast images having a phase difference of opposite sign upon interference of light beams for observation of an object. These two contrast images are from two defocus images which have been captured under microscopy with respect to a focus image of cell(s) by shifting the focal point in opposite directions along the optical axis.

Moreover, a method for measuring a thickness distribution of an object using a focus image and two defocus images has also been known (for example, refer to Australian Patent Application Publication No. 2004201109A1). A method for measuring a thickness distribution of an object using interference fringes resulting from interference between light which is transmitted through or is reflected from the object and reference light has also been known.

However, in the above method for measuring a thickness of an object optically, the difference between a reflective index of the object and a reflective index of the ambient environment is used, so that the above method readily comes under influence of alternation of the reflective index of the object and the reflective index of the ambient environment. In the case of applying the above method to a culture cellular thickness measurement, components of the culture solution containing cells in a culture vessel vary because cells absorb nutrients in the culture solution and excrete waste matters into the culture solution during culture. As components of the culture solution vary, the refractive index thereof is changed, which thus leads to a concern regarding difficulty in precise measurement of the cellular thickness distribution.

BRIEF SUMMARY OF THE INVENTION

The present invention takes the above situation into consideration with an object of providing a culture vessel and a cellular thickness measurement method capable of precisely measuring the cellular thickness distribution regardless of changes in the refractive index of a culture solution with the progress of culture.

In order to achieve the above object, the present invention provides the following solutions.

The present invention provides a culture vessel made of a transparent material with a culture surface capable of culturing cells in an adhesive manner on the bottom face, wherein a reference substance having an already known refractive index and an already known thickness dimension is fixed to a part of the culture surface.

According to the present invention, in a state where a culture solution is stored in such a way that the reference substance sinks under the culture solution, light of a predetermined wavelength is transmitted through the reference substance and the culture solution, and phase information of the detected light are detected, which thereby enables estimate of the refractive index of the culture solution on the basis of the phase information, the already known refractive index of the reference substance, and the already known thickness dimension thereof. Accordingly, regardless of changes in the refractive index of the culture solution due to absorption of nutrients and excretion of waste matters by cells with the progress of cell culture, thus changed refractive index of the culture solution can be readily estimated and the cellular thickness dimension can be precisely measured.

The present invention also provides a method for measuring a thickness dimension of a cell being cultured in a culture solution stored in such a way that the cell and the reference substance sink under the culture solution, in an adhesive manner onto the culture surface of the culture vessel, wherein the cellular thickness measurement method comprises:

a photographing step of capturing a phase information image which shows two-dimensional distribution of phase information by transmitting light of a predetermined wavelength through the cell, the reference substance, and the culture solution, and by photographing the transmitted light;
a refractive index calculation step of calculating a refractive index of the culture solution on the basis of the phase information at a position of the reference substance, the thickness dimension of the reference substance, and the refractive index thereof; and
a thickness calculation step of calculating the thickness dimension of the cell on the basis of the phase information at a position of the cell, and the refractive index of the culture solution that has been calculated in the refractive index calculation step.

According to the present invention, in the photographing step, an image including information of light having its phase changed in accordance with the refractive indexes of the cell and the culture solution is captured, by injecting light of a predetermined wavelength either downward or upward through the culture vessel made of a transparent material, and by detecting the light transmitted downward or upward through the culture vessel, the cell being cultured in an adhesive manner onto the culture surface of the culture vessel, and the culture solution covering thereover. By extracting the phase information from the image, the phase information image can be captured. Accordingly, in the refractive index calculation step, the refractive index of the culture solution can be calculated with use of the phase information at the position of the reference substance, the thickness dimension of the reference substance, and the refractive index thereof.

Subsequently thereafter, in the thickness calculation step, the cellular thickness dimension can be precisely calculated with use of the phase information at the position of the cell, the refractive index of the cell, and the calculated refractive index of the culture solution.

That is to say, the refractive index of the culture solution is calculated prior to the calculation of the cellular thickness dimension. Therefore, regardless of changes in the refractive index of the culture solution due to reduction in nutrients and increment of waste matters from cells in the culture solution with the progress of cell culture, the cellular thickness dimension can be precisely measured.

The present invention demonstrates an effect in which the cellular thickness distribution can be precisely measured regardless of changes in the refractive index of a culture solution with the progress of culture. Thus, the present invention also demonstrates an effect in which alternation of cellular shape can be observed in three dimensions and alternation of the cellular thickness distribution can be measured, while culture is continued.

BRIEF DESCRIPTION OF THE SEVERAL VIEWS OF THE DRAWINGS

FIG. 1 is a longitudinal cross-section showing a culture vessel according to one embodiment of the present invention.
FIG. 2 is an explanatory diagram of a cellular thickness dimension measurement method according to the present embodiment with use of the culture vessel of FIG. 1.
FIG. 3 is a graph showing the distribution of phase information taken along a straight line in a phase information image that has been captured by the measurement method of FIG. 2.
FIG. 4 is a flowchart showing the measurement method of FIG. 2.

DETAILED DESCRIPTION OF THE INVENTION

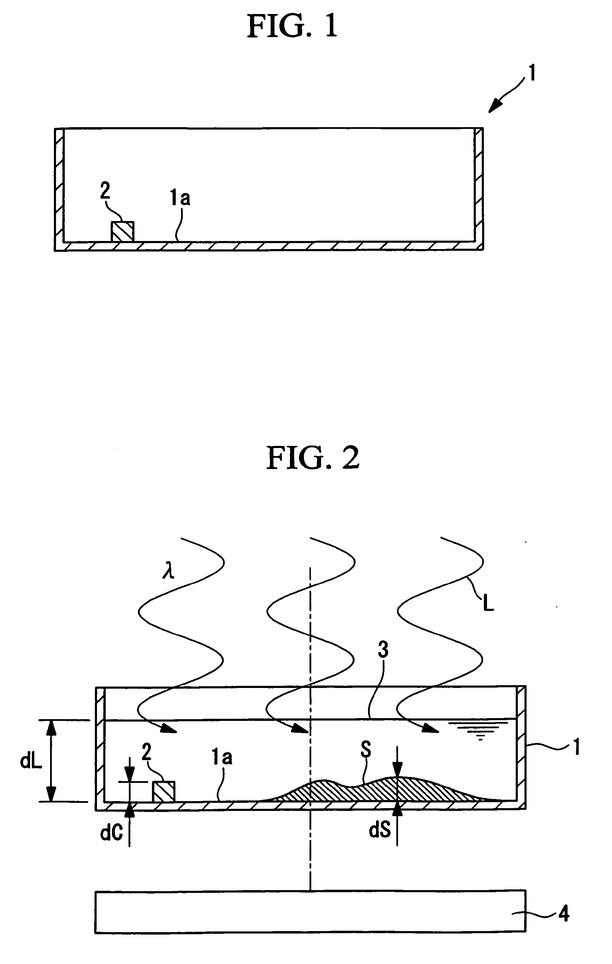

Hereunder is a description of a culture vessel and a cellular thickness measurement method according to one embodiment of the present invention, with reference to FIG. 1 to FIG. 4.
As shown in FIG. 1, a culture vessel 1 according to the present embodiment is a Petri dish-like container made of a transparent material with a culture surface 1a for culturing adhesive cells in an adhesive manner on the bottom face, wherein a reference substance 2 having an already known refractive index and an already known thickness dimension is fixed to at least a part of the culture surface 1a.

The material of the reference substance 2 is not limited as long as the substance has a uniform refractive index and an accurate thickness dimension, although it is preferably made of a biocompatible material such as polystyrene. Moreover, the reference substance 2 is adhered onto the culture surface 1a through an adhesive layer which is too thin to affect the measurement. The culture surface 1a around the reference substance 2 may be subjected to arrangement or treatment to avoid adhesion of cells.

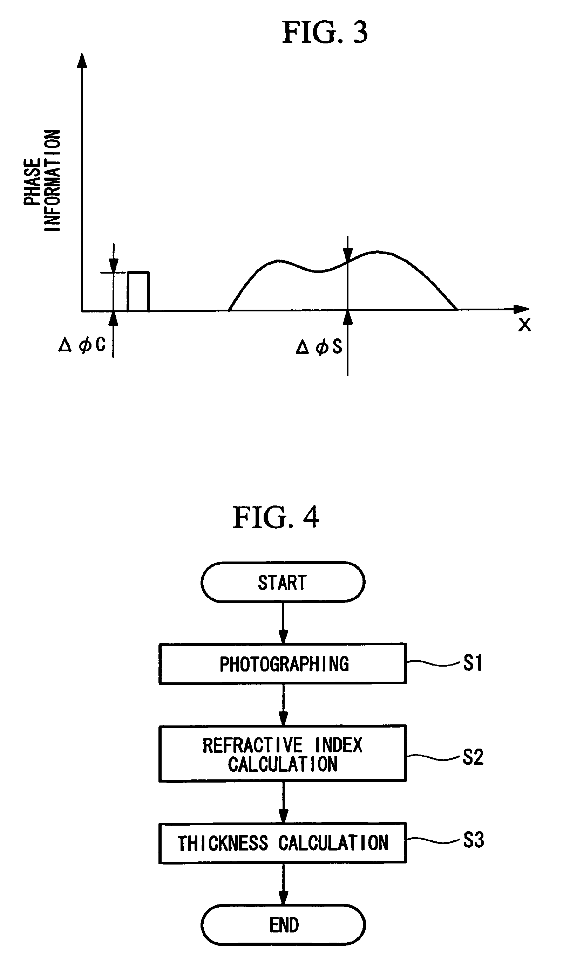

As shown in FIG. 4, the cellular thickness measurement method according to the present embodiment comprises: a photographing step S1; a refractive index calculation step S2 of calculating a refractive index nL of a culture solution 3; and a thickness calculation step S3 of calculating a cellular thickness dimension on the basis of the calculated refractive index nL of the culture solution 3.

The photographing step S1 is performed such that, as shown in FIG. 2: cells S to be measured are adhered onto the culture surface 1a of the culture vessel 1; light L of a predetermined wavelength is transmitted through the cells S, the reference substance 2, and the culture solution 3, at any point in time during the culturing process in which the culture solution 3 is stored to have a depth greater than the thickness dimensions of the cells S and the reference substance 2; and the transmitted light L is photographed by a two-dimensional imager 4 such as CCD. The photographing step S1 is designed to capture a phase information image which shows two-dimensional distribution of phase information, by a conventional method using a focus image and two defocus images.

The phase information image that has been captured in the photographing step S1 can be regarded as, for example, a one-dimensional distribution of masses of phase information as shown in FIG. 3, by extracting phase information taken along an arbitrary straight line passing through the reference substance 2 in that phase information image.

The refractive index calculation step S2 is designed to calculate the refractive index nL of the culture solution 3 with use of the phase information of the reference substance 2 and therearound.

Specifically, it is assumed that the depth of the culture solution 3 is dL, the refractive index of the culture solution 3 is nL, the thickness dimension of the reference substance 2 is dC, the refractive index of the reference substance 2 is nC, the wavelength of light to be transmitted is λ, the phase difference information at a position of the reference substance 2 is ΔΦC, and the phase information therearound is ΔΦL. At a position of the reference substance 2, the reference substance 2 accounts for the thickness dimension dC of the depth dL of the culture solution. At positions therearound, the culture solution 3 fills the full depth dL.

Accordingly, a position of the reference substance 2 and positions therearound have different refractive indexes depending on the thickness dimension dC of the reference substance 2. Therefore, a phase difference $\Delta\Phi1$ occurs in accordance with the difference in the refractive index $\Delta n1 = (nC-nL)$ and the thickness dimension dC. That is to say,

$$\Delta\Phi1 = \Delta n1 \times \lambda \times dC\ldots(1).$$

The refractive index nC of the reference substance 2, the light wavelength $\lambda$, and the thickness dimension dC of the reference substance 2 are already known, and thus the equation (1) is transformed such that:

$$nL = nC-\Delta\Phi1/(\lambda\times dC)\ldots(2).$$

The refractive index nL of the culture solution 3 can be calculated by substituting the phase difference $\Delta\Phi1$ which has been obtained by measurement, into the above equation.

The thickness calculation step S3 is similarly designed to calculate the thickness dimension of each position of the cells S with use of the phase information of the cells S and therearound.

Specifically, it is assumed that the depth of the culture solution 3 is dL, the refractive index of the culture solution 3 is nL, the thickness dimension of the cells S is dS, the refractive index of the cells S is nS, the phase information at a specific position of the cells S is $\Delta\Phi S$. At a position of the cells S, the cells S account for the thickness dimension dS of the depth dL of the culture solution 3. At positions around the cells S, the culture solution 3 fills the full depth dL.

Accordingly, a specific position of the cells S and positions around the cells S have different refractive indexes depending on the thickness dimension dS of the cells S. Therefore, a phase difference $\Delta\Phi2$ occurs in accordance with the difference in the refractive index $\Delta n2 = (nS-nL)$ and the thickness dimension dS. That is to say,

$$\Delta\Phi2 = \Delta n2 \times \lambda \times dS\ldots(3).$$

The light wavelength $\lambda$ is already known, and with use of the refractive index nL of the culture solution 3 that has been obtained by the equation (2), the refractive index nS of the cells S that has been determined by separate measurement, and the phase difference $\Delta\Phi2$ that has been obtained by measurement, the thickness dimension dS of the cells S can be obtained by the equation (4):

$$dS = \Delta\Phi2 / (\lambda\times\Delta n2)\ldots(4).$$

In this way, according to the culture vessel 1 of the present embodiment, the reference substance 2 is used to measure the refractive index nL of the culture solution 3, and the measured refractive index nL of the culture solution is used to measure the thickness dimension of the cells S. Accordingly, an advantage is provided in which, regardless of changes during culture in the refractive index nL of the culture solution 3 due to absorption of nutrients and excretion of waste matters by the cells S with the progress of culture, thus changed refractive index of the culture solution nL can be readily measured and the thickness dimension of the cells S can be precisely measured.

In the present document, a method using a focus image and a defocus image has been explained as a method for capturing the phase information image, but the present invention should not be limited to the method using a focus image and a defocus image. A phase information capturing means using an interference fringes between transmitted light and reference light may be available.

**Claims**

1. A culture vessel made of a transparent material with a culture surface capable of culturing cells in an adhesive manner on the bottom face, wherein a reference substance having an already known refractive index and an already known thickness dimension is fixed to a part of the culture surface.

2. A method for measuring a thickness dimension of a cell being cultured in a culture solution stored to have a depth greater than the thickness dimensions of the cell and the reference substance, in an adhesive manner onto the culture surface of the culture vessel according to claim 1, wherein the cellular thickness measurement method comprises:

   a photographing step of obtaining two-dimensional distribution of phase information by transmitting light of a predetermined wavelength through the cell, the reference substance, and the culture solution, and by photographing the transmitted light;
   a refractive index calculation step of calculating a refractive index of the culture solution on the basis of the phase information at a position of the reference substance, the thickness dimension of the reference substance, and the refractive index thereof; and

a thickness calculation step of calculating the thickness dimension of the cell on the basis of the phase information at a position of the cell, a refractive index of the cell, and the refractive index of the culture solution that has been calculated in the refractive index calculation step.

# FIG. 1

# FIG. 2

# FIG. 3

# FIG. 4

Europäisches
Patentamt

European
Patent Office

Office européen
des brevets

**EUROPEAN SEARCH REPORT**

Application Number

EP 08 01 7582

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2005/059092 A (CANON KK [JP]; KANOME OSAMU [JP]; WATANABE KOHEI [JP]; MIYAZAKI TAKESH) 30 June 2005 (2005-06-30)<br>* page 11, lines 7-13; figure 1 *<br>* page 14, lines 7-9 *<br>* page 17, line 22 - page 18, line 27 * | 1 | INV.<br>C12M1/22<br>C12M1/34<br>G01B11/06<br>G01N21/41 |
| Y | | 2 | |
| Y | CURL C L ET AL: "Single Cell Volume Measurement by Quantitative Phase Microscopy (QPM) : A Case Study of Erythrocyte Morphology"<br>CELLULAR PHYSIOLOGY AND BIOCHEMISTRY, KARGER AG, BASEL, CH,<br>vol. 17, 1 January 2006 (2006-01-01), pages 193-200, XP002511706<br>* page 195 * | 2 | |
| X | EP 0 751 215 A (BECTON DICKINSON CO [US]) 2 January 1997 (1997-01-02)<br>* abstract; figure 2 *<br>* column 5, line 57 - column 6, line 17 * | 1 | |
| | | | TECHNICAL FIELDS SEARCHED (IPC) |
| A | BARER R: "Refractometry and Interferometry of Living Cells"<br>JOURNAL OF THE OPTICAL SOCIETY OF AMERICA,,<br>vol. 47, no. 6, 1 June 1957 (1957-06-01), pages 545-556, XP002511707<br>* page 555, right-hand column * | 2 | C12M<br>G01B<br>G01N |
| A | WO 03/012407 A (IATIA IMAGING PTY LTD [AU]; ALLMAN BRENDAN EDWARD [AU]; NUGENT KEITH [] 13 February 2003 (2003-02-13)<br>* the whole document * | 2 | |

-/--

The present search report has been drawn up for all claims

| Place of search | Date of completion of the search | Examiner |
|---|---|---|
| Berlin | 27 January 2009 | Brison, Olivier |

CATEGORY OF CITED DOCUMENTS

X : particularly relevant if taken alone
Y : particularly relevant if combined with another
      document of the same category
A : technological background
O : non-written disclosure
P : intermediate document

T : theory or principle underlying the invention
E : earlier patent document, but published on, or
      after the filing date
D : document cited in the application
L : document cited for other reasons

& : member of the same patent family, corresponding
      document

EPO FORM 1503 03.82 (P04C01)

## EUROPEAN SEARCH REPORT

**Application Number**

EP 08 01 7582

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | CURL CLAIRE L ET AL: "QUANTITATIVE PHASE MICROSCOPY: A NEW TOOL FOR MEASUREMENT OF CELL CULTURE GROWTH AND CONFLUENCY IN SITU"<br>PFLUEGERS ARCHIV: EUROPEAN JOURNAL OF PHYSIOLOGY, SPRINGER VERLAG, BERLIN, DE, vol. 448, no. 4,<br>17 February 2004 (2004-02-17), pages 462-468, XP008074000<br>ISSN: 0031-6768<br>* page 464, left-hand column *<br>----- | 1,2 | |
| A | CURL C L ET AL: "Quantitative Phase Microscopy : A New Tool for Investigating the Structure and Function of Unstained Live Cells"<br>CLINICAL AND EXPERIMENTAL PHARMACOLOGY AND PHYSIOLOGY,,<br>vol. 31, 1 January 2004 (2004-01-01), pages 896-901, XP002511710<br>* page 900 *<br>----- | 1,2 | |
| | | | TECHNICAL FIELDS SEARCHED (IPC) |

The present search report has been drawn up for all claims

| Place of search | Date of completion of the search | Examiner |
|---|---|---|
| Berlin | 27 January 2009 | Brison, Olivier |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 08 01 7582

This annex lists the patent family members relating to the patent documents cited in the above-mentioned European search report.
The members are as contained in the European Patent Office EDP file on
The European Patent Office is in no way liable for these particulars which are merely given for the purpose of information.

27-01-2009

| Patent document cited in search report | | Publication date | Patent family member(s) | | Publication date |
|---|---|---|---|---|---|
| WO 2005059092 | A | 30-06-2005 | JP | 2005176630 A | 07-07-2005 |
| | | | US | 2007259394 A1 | 08-11-2007 |
| EP 0751215 | A | 02-01-1997 | CA | 2180082 A1 | 30-12-1996 |
| | | | JP | 9117277 A | 06-05-1997 |
| WO 03012407 | A | 13-02-2003 | NONE | | |

EPO FORM P0459

For more details about this annex : see Official Journal of the European Patent Office, No. 12/82

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2005218379 A

- AU 2004201109 A1